# EUROPEAN PATENT APPLICATION

(11) **EP 3 851 186 A1**
(43) Date of publication of application: **21.07.2021**
(21) Application number: 20152249.7
(22) Date of filing: 16.01.2020
(51) Int. Cl.: B01J 13/16, A01N 25/28, A23P 10/30, A61K 8/11, A61K 9/48, C11D 3/50, D06M 23/12

(54) **SUBMICRON PUU-CAPSULES AND MANUFACTURING THEREOF**

(71) Applicant: EMPA Eidgenössische Materialprüfungs- und Forschungsanstalt, 8600 Dübendorf (CH)
(72) Inventor: LUISIER, Nicolas, 9012 St. Gallen (CH); FORTUNATO, Giuseppino, 9000 St. Gallen (CH); TONCELLI, Claudio, 9014 St. Gallen (CH)
(74) Representative: Grimm, Siegfried

(57) **Abstract**

The present invention relates to microcapsules comprising a Poly-(Urethane-Urea) wall encapsulating an active ingredient, to compositions comprising such microcapsules and to textiles functionalized with such microcapsules. The inventive microcapsules are obtainable by a process combining ultrasonication, interfacial polymerisation and curing steps.

## Description

The present invention relates to microcapsules comprising a Poly-(Urethane-Urea) wall encapsulating an active ingredient, to compositions comprising such microcapsules and to textiles functionalized with such microcapsules. The inventive microcapsules are obtainable by a process combining ultrasonication, interfacial polymerisation and curing steps.

It is generally accepted that one of the problems faced by the textile industry lies in the relatively rapid loss of olfactive benefit provided by fragrances due to their volatility. In order to tailor the release rates of fragrances, delivery systems such as microcapsules containing a fragrance, are developed to protect and later release the core payload. A key requirement from the industry regarding these systems is to survive suspension in challenging conditions without physically dissociating or degrading. This is referred to as performance in terms of stability for the delivery system. Further, functionalized textiles have to comply with a number of requirements, such as: compatibility with the skin, compatibility with existing impregnation processes, cost considerations, stability and release performance.

The majority of the commercially available microencapsulated fragrance systems with interest for textile applications are based on phenol-formaldehyde / melamine-formaldehyde resins. Recognized human health problems concerning formaldehyde emissions led to the use of poly(urethane-urea) (PUU) systems.

Silva at al. (The Journal of the Textile Institute, Vol.108, No.3, 361-367) describe PUU/limonene microcapsules produced by interfacial polymerisation. According to the authors, the microcapsules tend to agglomerate, requiring the presence of an emulsifier. The document further describes impregnation of textiles with such microcapsules. Although the authors spend much effort on the impregnation, including an optimized conditions and pre-treatment of the capsules (p.363, left col.), only insufficient coating is observed (fig. 6). The authors also realize that some microcapsules are broken and emptied-out (p.364, right col.). Consequently, the performance of the impregnation requires improvement, indicated by 5- 20% retained limonene after one dry washing (fig.8).

Thus, it is an object of the present invention to mitigate at least some of these drawbacks of the state of the art. In particular, it is an aim of the present invention to provide alternative, particularly improved microcapsules useful to impregnate textiles.

There is also an ever-existing need to provide alternative and/or improved methods for manufacturing microcapsules and impregnated textiles.

These objectives are achieved by the capsule composition as defined in claim 1 and the manufacturing method as defined in claim 8. Further aspects of the invention are disclosed in the specification and independent claims, preferred embodiments are disclosed in the specification and the dependent claims.

The present invention will be described in more detail below. As used herein, the term "a", "an", "the" and similar terms used in the context of the present invention (especially in the context of the claims) are to be construed to cover both the singular and plural unless otherwise indicated herein or clearly contradicted by the context. As used herein, the terms "containing" shall include the meaning "consisting of", "essentially consisting of" and "comprising". Further, specific embodiments and definitions may be combined at will. Specifically, definitions given in the context of manufacturing methods shall also apply to materials, such as capsules and textiles, and vice versa, unless otherwise indicated or clearly contradicted by the context.

The present invention will be better understood by reference to the **figures.**

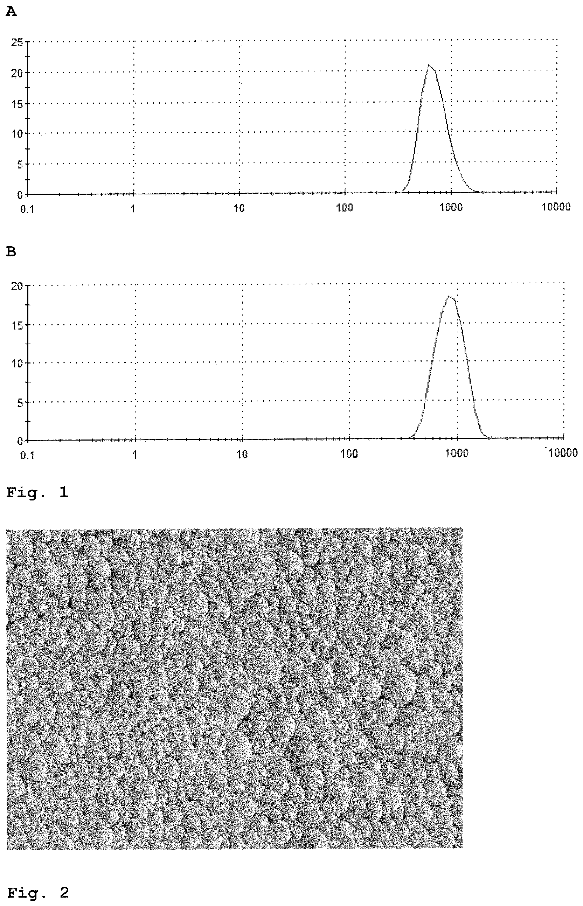

**Figure 1****:** Size distribution by number and by volume of microcapsules from example 1 as determined by dynamic light scattering. X-axis represents the diameter in nm and Y-axis represents the number in % (A) and volume in % (B), respectively.
**Figure 2****:** Scanning electron micrograph of microcapsules from example 1. Magnification is 10'000x.

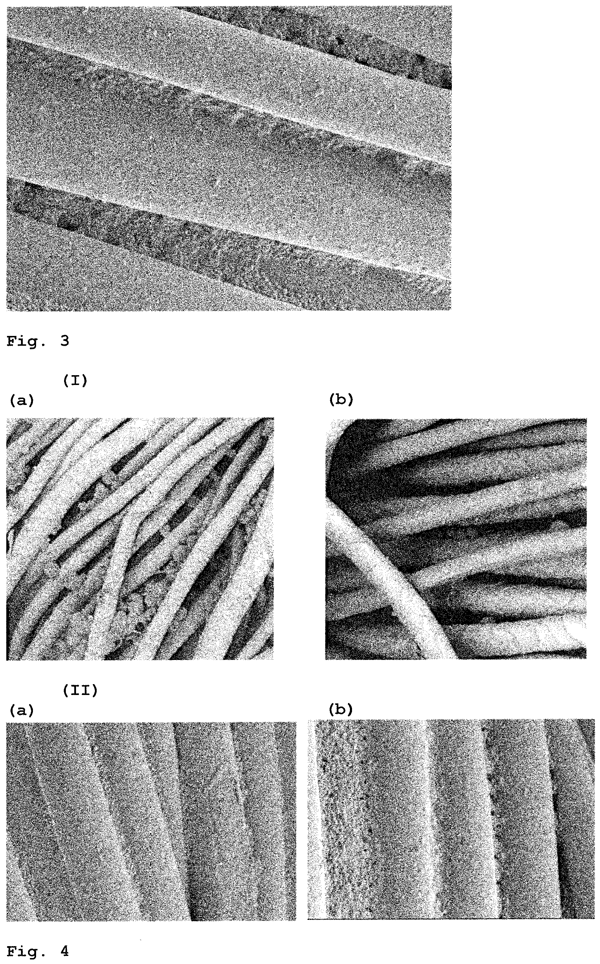

**Figure 3****:** Scanning electron micrograph of impregnated textile from example 2. Magnification is 2'000x.
**Figure.4****:** Optical microscopy of impregnated textile (I), for comparison (Silva et al, Fig. 9, cited above): microcapsules before (a) and after (b) 1 washing cycle. It is apparent that most of the known microcapsules according to Silva et al (I) are lost after one washing cycle. (II) This invention: microcapsules of example 2 on polyester fibers before (a) and after (b) 1 washing cycle. It is apparent that the inventive microcapsules show a significant improved adherence to the textile.

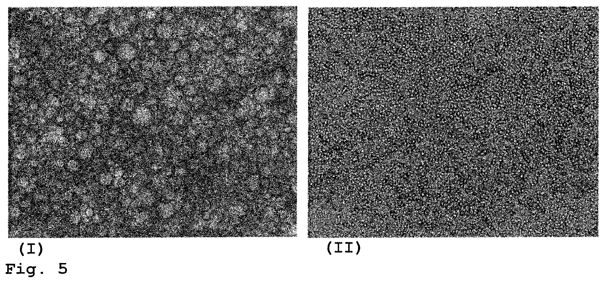

**Fig. 5****:** Optical microscopy of capsule composition, Magnification is 400x for both
   (I) for comparison (Silva et al, Fig. 4, cited above)
   (II) inventive capsules, example 1.
   It is apparent, that the inventive capsules are significantly smaller with unimodal size distribution.

In more general terms, in a **first aspect,** the invention relates to a capsule composition comprising a plurality of poly (urethane-urea) microcapsules ("PUU-microcapsules"), wherein the PUU microcapsules contain an encapsulated active ingredient and a poly (urethane-urea) wall ("PUU-wall"); and wherein the PUU-microcapsules have a sub-micron size and a unimodal size distribution.
It was found that the inventive compositions show improved properties when compared to known capsule compositions. For example, the inventive capsule compositions show an improved release profile for active ingredients, particularly long lasting properties. Further, they are simple in application on fabrics / textiles, complying with international environmental standards (such as Oekotex). Still further, the capsules are safe when used, no adverse effects on the skin are observed. Still further, the capsules are compatible with a wide range of active ingredients, no limitation towards specific functional groups, such as -OH incompatibility, was observed. Without being bound to theory, it is believed that the combination of submicron-sized particles with the PUU wall provides for particularly suitable compositions to reliably encapsulate a broad range of active ingredients and to impregnate a broad range of fabrics.

This aspect of the invention shall be explained in further detail below:
**Morphology of Capsules:** An important aspect of this invention is the morphology of microcapsules, which may be characterized by one or more of the parameters size, size distribution, shell thickness.
The inventive capsules are of sub-micron size, preferably 20 nm - 1500 nm, more preferably 50 nm - 1000 nm, most preferably 300 nm - 900 nm. The inventive capsules show a unimodal size distribution, typically with a FWHM of 100-800 nm, preferably of 300-500 nm. Size and size distribution may be determined by dynamic light scattering. Until now, it was not possible to obtain capsules having such sub-micron and having a unimodal size distribution. Such capsules are now obtainable according to the manufacturing method described herein (c.f. 2^{nd} aspect of the invention). Without being bound to theory, it is believed such size and size distribution contributes to the beneficial properties of the inventive capsules. Advantageously, the shell thickness is 8-50 nm, preferably 8 - 16 nm, such as approximately 10 nm.
The capsule compositions show good physical / mechanical properties and safely encapsulate the active ingredients. This is an important feature to allow coating of fabrics / textiles. Nevertheless, the capsule compositions allow a slow release of the active ingredient. This is an important feature to perform its task as a vehicle for delivering / releasing the active ingredient. The combination of both contradicting features, mechanical stability and suitable release profile is achieved with the present capsule composition.

**Active Ingredient:** A broad range of active ingredients may be encapsulated by the inventive microcapsules. In view of the encapsulating material, the PUU-wall, hydrophobic active ingredients are preferred. In the context of this invention, an active ingredient is considered hydrophobic, if it forms a two-phases dispersion when mixed with water. Suitable active ingredient may be selected from the group consisting of fragrances (or perfumes), malodour counteracting agents, repellents, fungicides, bactericides, pharmaceutically active ingredients, cosmetics, and food additives. Such active ingredients are known per se and commercially available. The term active ingredient includes a single active ingredient (e.g. a repellent), a mixture or combination of active ingredients (e.g. a fragrance composition), in each case optionally diluted with a solvent.

The active ingredient (particularly a perfume composition) may be dissolved in a solvent of current use in the perfume industry. Examples of suitable solvents include diethyl phthalate, isopropyl myristate, Abalyn® (rosin resins, available from Eastman), benzyl benzoate, ethyl citrate, limonene or other terpenes, or isoparaffins. Preferably, the solvent is very hydrophobic and highly sterically hindered, like for example Abalyn®. Preferably the active ingredient comprises less than 30%, preferably less than 10% of solvent. In an embodiment the invention, the solvent is not an alcohol. In an embodiment of the invention, the active ingredient is essentially free of, or free of, solvent.

The nature and type of repellent may be chosen by the skilled person in view of the intended application. Examples of repellents include birch, DEET (N,N-diethyl-m-toluamide), essential oil of the lemon eucalyptus (Corymbia citriodora) and its active compound p-menthane-3,8-diol(PMD), icaridin (hydroxyethyl isobutyl piperidine carboxylate), Nepelactone, Citronella oil, Neem oil, Bog Myrtle (Myrica Gale), Dimethyl carbate, Tricyclodecenyl allyl ether, 1R3535 (3-[N-Butyl-N-acetyl]-aminopropionic acid, ethyl ester, Ethylhexanediol, Dimethyl phthalate, Metofluthrin, Indalone, SS220, anthranilate-based insect repellents, and mixtures thereof.

The nature and type of fragrance may be chosen by the skilled person in view of the intended application. Fragrances may belong to chemical classes as varied as alcohols, aldehydes, ketones, esters, ethers, acetates, nitriles, terpenoids, nitrogenous or sulphurous heterocyclic compounds and essential oils. Fragrances may be of natural or synthetic origin. A specific example of a fragrance is limonene.

**PUU Wall:** The inventive microcapsules comprise a PUU wall, surrounding the active ingredient. The PUU wall contains, typically consists of, a urethane-urea-polymer ("PUU"). The polymer may or may not be cross-linked. Advantageously, the PUU is a crosslinked PUU. Such polymers are known per se and described in the prior art cited above. It is apparent that PUU distinguishes from Polyurethanes ("PU" or "PUR", the reaction product of diisocyanates and diols) and from Polyureas (the reaction product of diisocyanates and diamines).

Typically, such urethane-urea-polymers are characterized by its starting materials: one or more diisocyanates, one or more polyols, one or more curing agents with aminogroups and optionally one or more crosslinkers. These starting materials are commercial items and / or available according to known methods.

The diisocyanate component includes aromatic diisocyanates and aliphatic diisocyanates and combinations thereof.
The term "aromatic diisocyanate" includes any diisocyanate comprising an aromatic moiety.
In an embodiment of the invention, the term diisocyanate comprises a phenyl, a toluyl, a xylyl, a naphthyl or a diphenyl moiety, more preferably a toluyl or a xylyl moiety.
In embodiments of the invention, the aromatic diisocanate is selected from the group consisting of a polyisocyanurate of toluene diisocyanate (commercially available from Bayer under the tradename Desmodur® RC), a trimethylol propane-adduct of toluene diisocyanate (commercially available from Bayer under the tradename Desmodur L75), a trimethylol propane-adduct of xylylene diisocyanate (commercially available from Mitsui Chemicals under the tradename Takenate® D-110N).
In embodiments of the invention, the aromatic diisocyanate is selected from the group consisting of Methylene diphenyl diisocyanate (MDI), Toluene diisocyanate (TDI), Lupranat® 102 (from BASF) and Lupranat® 105 (from BASF).

The term "aliphatic polyisocyanate" is defined as a polyisocyanate which does not comprise any aromatic moiety. In embodiments of the invention, the aliphatic diisocyanate is selected from the group consisting of Isophorone diisocyanate (IPDI) 4,4'-Methylenebis(cyclohexyl isocyanate) and Hexamethylene diisocyanate.

The polyol component includes polyethylene glycols and cyclic diols. In one embodiment, the polyethylene glycol is selected from the group consisting of PEG's 200 - > 5000, preferably PEG's 200 -> 1000, such as PEG 400. In one embodiment, the cyclic diol is isosorbide.

The curing agent includes polyamines, preferably diamines. In one embodiment, the diamine is selected from the group consisting of hydrazine and ethylenediamine, preferably hydrazine or its hydrate.

The crosslinking component, if present, includes tri- and / or tetra-functional compounds with reactive hydroxy or amino groups. It is preferred to include crosslinking components. Suitable crosslinkers may be selected from the group consisting of triols, tetraols and polyamines. In one embodiment, the crosslinker is selected from the group consisting of Trimethylol propane, Pentaerythritol, Tris(aminoethyl) amine, and Branched polyethyleneimine.

PUU formation is typically induced by a catalyst. As a consequence thereof, the PUU wall may contain a catalyst. Suitable catalysts are known in the field and include organic catalysts such as DABCO, DBU, Dimethylethanolamine and metalorganic catalysts, such as Dibutyltindilaurate and Bismuthneodecanoate.

The ratio of the starting materials may be determined by routine experiments to allow full polymerization.

**Capsule Characteristics.** Suitably, the inventive microcapsules comply with one or more of the following characteristics. These are obtained when following the manufacturing described below.

In an advantageous embodiment, the microcapsules have a fill factor (number of capsules containing active ingredients / total number of capsules) above 80%, preferably above 90%. Obtaining such a high fill factor is an advantageous property of the inventive microcapsules, observed when following the method described herein.

In an advantageous embodiment, the microcapsules have a high resistance to abrasion. Obtaining such high resistance is an advantageous property of the inventive microcapsules, observed when following the method described herein.

In an advantageous embodiment, the microcapsules have a low residual -NCO monomer content, such as a residual -NCO monomer content < 0.1 %, preferably below the detection limit. Obtaining such low concentration is an advantageous property of the inventive microcapsules, observed when following the method described herein and particularly relevant for the application on textiles as described below. The residual monomer content may be determined by GC-MS or FT-IR.

In an advantageous embodiment, the microcapsules present a linear release profile, such as the smell detected by the end-user is constant over time.

**Capsule Composition:** It is apparent from the above that not one single microcapsule is obtained, but a plurality of such microcapsules. In one embodiment the capsule composition is present in the form of a slurry, said slurry preferably comprising the inventive microcapsules dispersed in an aqueous dispersing medium. Such slurries include the direct reaction product of the inventive process described herein ("crude product"), as well as purified and re-dispersed microcapsules ("purified slurry"). Accordingly, the aqueous dispersing medium may contain, in addition to water, non-reacted starting materials, catalyst, surfactants, stabilizing agents, pH modifying agents.
Suitable surfactants may be selected by the skilled person and include commercial products such as Tween 20, Tween 80, Span 20, Span 80, Lutensol, SDS, Triton X-100, and CTAB.
Suitable stabilizing agents may be selected by the skilled person and include commercially available stabilizers, such as from the class of poloxamers, of polyvinyl alcohols, of polyacrylic acids, carrageenanes.

In one embodiment the capsule composition is present in the form of a dried powder. It was found that the dried microcapsules are stable and may be re-dispersed without detrimentally influencing its properties. This is an important advantage of the inventive microcapsules.

In a **second aspect,** the invention relates to a process for manufacturing a capsule composition as described herein, comprising the step of preparing an oil-in-water emulsion with droplet size of less than 1500 nm with the aid of ultrasonication, the oil phase containing active ingredient and diisocyanate (Step (b) below), causing formation of microcapsules by interfacial polymerisation with polyol, catalyst and optionally crosslinker (step (c) below) and curing the thus obtained microcapsules with a curing agent (step (d) below) to obtain the inventive PUU microcapsules in the form of a slurry. This process allows for manufacturing new capsule compositions with improved properties as described herein. Specifically, it solves the problem of providing highly stable capsules having at the same time a beneficial release profile for the active ingredient. The present solution to this problem, the process described herein, is not described or even suggested in any prior art document.

In one embodiment, the invention provides for a process for preparing a capsule composition as described herein, said process comprising the steps of (a) preparing the following starting materials: (i) an oil phase comprising a hydrophobic active ingredient, a diisocyanate monomer and optionally a diluent ("oil phase"); (ii) a 1^{st} aqueous phase, not miscible with said organic phase, comprising water and a stabilizer; (iii) a 2^{nd} aqueous phase comprising water, a polyol, optionally a crosslinker and a catalyst; (iv) a 3^{rd} aqueous phase, comprising water and a curing agent; (b) combining oil phase and 1^{st} aqueous phase to obtain an oil-in-water dispersion with droplet size of more than 1500 nm followed by ultrasonic treatment to obtain an oil-in-water emulsion with droplet size of less than 1500 nm; (c) combining the thus obtained oil-in-water emulsion with said 2^{nd} aqueous phase and applying conditions sufficient to induce interfacial polymerisation to obtain an initial capsule composition in form of a slurry (said capsules having an encapsulated active ingredient and a particles size of 20 - 1500 nm and unimodal size distribution); and (d) combining the thus obtained initial capsule composition with said 3^{rd} aqueous phase under conditions sufficient to induce curing of non-reacted isocyanate groups to obtain the capsule composition in the form of an aqueous emulsion.

In one further embodiment, the invention provides for a process for preparing a capsule composition as described herein comprising as additional steps: (e) separating the capsule composition from the aqueous dispersing medium; (f) optionally purifying the capsule composition followed by either (g) or (h); (g) re-suspending the capsule composition in a 5^{th} aqueous phase, preferably water, to obtain a slurry; or (h) drying the capsule composition to obtain a dried powder.

This aspect of the invention, particularly process steps (a) to (h) shall be explained in further detail below:
**Step (a):** Preparing starting materials, oil phase, 1^{st} ... 5^{th} aqueous phase is entirely conventional in the field. Advantageously, all starting materials are present in the form of a solution. The components of the starting materials are discussed above, first aspect of the invention.
The 1^{st} aqueous phase typically contains water and an emulsion stabilizer. Suitable stabilizers are known in the field and non-reactive towards the remaining components. Examples of stabilizers are polyvinyl alcohol, cellulose derivatives such as hydroxyethyl cellulose, polyethylene oxide, copolymers of polyethylene oxide and polyethylene or polypropylene oxide, copolymers of acrylamide and acrylic acid or cationic polymers such as for example a cationic copolymer of vinylpyrrolidone and of a quaternized vinylimidazole such as those sold under the trade name Luviquat® (commercially available from BASF). Preferably, the colloidal stabilizer is polyvinyl alcohol or a cationic polymer, which is a copolymer of vinylpyrrolidone and of a quaternized vinylimidazole, or a mixture thereof. Particularly preferred stabilizers are Polyvinylalcohols.

**Step (b):** The emulsification step is an important element of the inventive process, as it influences the properties of the capsule composition significantly. It comes as a surprise that this step has an important effect on performance in general and to abrasion resistance in particular. Typically, emulsification is performed in two consecutive emulsification steps (b1) and (b2). In (b1), an emulsion is prepared with droplet size of more than 1500 nm. This may be achieved by high shear mixing with more than 10 000 rpm. In (b2), droplet size is reduced to less than 1500 nm. This is achieved by ultrasonic treatment with known instruments, e.g. available from Bronson Ultrasonics Corp. Suitable instruments typically have 400 W power, and are operated at 50 % amplitude or more in "pulsed" mode. In embodiments of the invention, instruments are operated at 80 % amplitude. Suitable treatment times are more than 1 minute, such as more than 5 minutes, e.g. 12 minutes.

**Step (c):** Parameters to cause interfacial polymerisation of polyisocyanate and polyol (and optionally cross-linker) are known in the field. The PU wall of the capsules is the result of the interfacial polymerisation between the polyisocyanate obtained in step (b) and the polyol added in step (c). Suitably, polymerisation takes place at temperatures at 40 - 100°C for 0.5-5 hrs. Catalyst concentration is determined by routine experiments.

**Step (d):** Parameters to cause curing are known in the field. The curing step ensures conversion of non-reacted isocyanate groups to urea groups, thereby forming the poly (urea-urethane) polymer wall of the capsules. Suitably, curing takes place at 40 - 100°C for 0.5-10 hrs.

**Step (e):** Due to the stability of the inventive microcapsules, separation from the dispersing medium is straightforward. Methods known in the field may be applied. Suitably is centrifugation, eg. at 35 000 xg.

**Step (f):** Purification steps are well established in the field. Washing steps, using an aqueous composition comprising water and optionally a surfactant ("4^{th} aqueous composition"), may be applied. Suitable surfactants may be selected from the group of non-ionic surfactants and include Pluronic F68.

**Step (g):** Re-dispersion steps are well established in the field. Such steps, using an aqueous composition comprising water and optionally additives ("5^{th} aqueous composition"), may be applied. Suitable additives may be selected in view of the intended use. It is an advantage of the inventive composition that re-dispersion is possible without, or almost without, coagulation.

**Step (g):** Drying steps are well established in the field. It is an advantage of the inventive composition that the inventive capsule composition can be converted to a dry product and may be used later without, or almost without, losing its beneficial properties.

Further, the invention also relates to a capsule composition obtainable by or obtained by a process as described herein. Accordingly, the invention provides for PUU- submicron-sized capsules containing hydrophobic active ingredients made via interfacial condensation (particularly of isocynanate and polyol), crosslinking (particularly with triols, tetraols or triamines), curing (particularly with polyamine).

In a **third aspect,** the invention relates to the use of capsule compositions as described herein in textile applications. In the context of this invention, textile applications shall include clothing (such as garments, sportwear and accessories), interior devices for home and transportation (such as curtains, carpets, and seat covers), food packaging and medical textiles (such as wound dressings, cosmetics, wellness textiles, wipes). As outlined above, the inventive capsule compositions comprise an active ingredient, having beneficial effects on textiles. For example, no adverse effects on the skin are observed and compatibility with oeko-tex standard 100 is achieved. Also, a stable release profile for the active ingredient is observed.

This aspect of the invention shall be explained in further detail below:
**Coated fabrics:** The capsule compositions are suited to impregnate all types of fabrics, including woven fabrics, non-woven fabrics, and knitted fabrics. Impregnation process may be achieved using standard methods, such as padding (using a foulard; or slope padding), coating (including roll-to-roll coating, reverse coating, gravure coating), spraying or immersion. Fixation of the inventive microcapsules may be ensured via gluing (as in example 2) or alternatively by covalent bonding or ionic bonding. The fixation of choice depends on the capsule and fabric type, as well as the equipment used. The skilled person is in a position to select appropriate impregnation methods.

Impregnation process may take place in the presence of a binder, for example a self-crosslinking binder, such as a resin of acrylic, polyurethane, silicone-type or starch. Such binders assist to glue the microcapsules onto the fabric, keeping them in place and thus preventing their loss during washing and wear. It was found that the inventive capsule compositions may be directly used in such impregnation process. There is no need for a pre-treatment, such as de-agglomeration by ultrasonication. The invention thus provides for a woven or non-woven or knitted fabric impregnated with a capsule composition as described herein. The impregnation does not modify the fabric's touch sensation or colour; no adverse effects to the skin are observed.

**Textiles:** The invention further provides for a textile product comprising the coated fabric described herein. As used herein, the term textile product is used in its broad sense and includes clothing (including sportswear and accessories), interior devices for home and transportation (such as curtains, carpets, and seat covers), food packaging and medical textiles (such as wound dressings, cosmetics, wellness textiles, wipes).

When using the inventive textiles, no toxicological issues are observed. The textiles are compatible with oeko-tex standard 100.

Textiles functionalized with the inventive capsule composition continuously emit fragrances (or other active ingredients) perfume over extended period of time, such as more than > 6 months. Further, textiles functionalized with the inventive capsule composition resist standard laundry operation in domestic conditions, such as 40°C standard washing program with detergent.

To further illustrate the invention, the following **examples** are provided. These examples are provided with no intend to limit the scope of the invention.

### Example 1 Synthesis of microcapsules (IPDI / PEG400 / trimethylol propane)

An organic phase was first formed by mixing a 1:1 mixture of glyceryl trioctanoate and limonene (12 mL) and isophorone diisocyanate (4 mL). A first aqueous phase was prepared from deionized (DI) water (100 mL) and PVA (Mowiol 4-88, 1.68 g). A miniemulsion was prepared by mixing these two solutions, emulsifying with a high-shear homogeneizer at 12'000 rpm for 3 minutes and ultrasonication (80% amplitude, pulsed, 12 min). In parallel, a second aqueous phase made from DI water (40 mL), PEG400 (8.2 mL), trimethylol propane (270 mg) and dibutyltin dilaurate (0.4 mL) was prepared separately. The miniemulsion was transferred to a 250 mL two-necks round bottom flask equipped with an overhead mechanical stirrer and the second aqueous phase was then added to it. The reaction was heated to 55 °C under constant mechanical agitation (150 rpm) for 2h. Then, a third solution of hydrazine hydrate (1.2 mL) in DI water (8 mL) was added dropwise and the mixture was let to react two more hours. The reaction was then cooled down to r.t, and the capsules were recovered by ultracentrifugation at 35'000 xg. Washing was performed by re-suspending the capsules in 1 % aqueous Pluronic F68 solution and the centrifugation step was repeated two times. Finally, the capsules were re-suspended in DI water for storage.

### Example 2 Impregnation of capsules on textile Impregnation:

Liquor solutions for foulard coating were prepared by mixing the binder polymer solution (30 mL, 150 g/L, Arristan CPU (a modified polyurethane, self-crosslinking upon drying) or Ecoperl HC (a modified polysiloxane, self-crosslinking upon drying) and the capsules of example 1 (12 g, 60 g/L) in DI water (170 mL). Textiles pieces of polyester (30 x 20 cm) were then coated in a laboratory foulard machine wherein the samples are first immersed in the coating liquor described above, and then the excess suspension is squeezed out by two rubber rollers. The coating process is performed at room temperature, the rollers speed is 2 m/min, the wet pick up controlled > 80% in weight and the samples are dried at air.

### Test results:

- The impregnated textiles release limonene for a prolonged period of more than 6 months.
- The impregnated textiles resist a standard laundry operation in domestic conditions @ 40°C standard washing program with detergent.
- Similar results are observed when replacing Polyester with cotton or silk.

### Example 3 Synthesis of microcapsules (MDI / PEG400 / trimethylol propane)

An organic phase was first formed by mixing limonene (12 mL) and methylene diphenyl diisocyanate (3.86 mL). A first aqueous phase was prepared from deionized (DI) water (100 mL) and PVA (Mowiol 4-88, 1.68 g). A miniemulsion was prepared by mixing these two solutions, emulsifying with a high-shear homogeneizer at 12'000 rpm for 3 min and ultrasonication (80% amplitude, pulsed, 12 min). In parallel, a second aqueous phase made from DI water (40 mL), PEG400 (8.2 mL), trimethylol propane (270 mg) and DABCO (280 mg) was prepared separately. The miniemulsion was transferred to a 250 mL two-necks round bottom flask equipped with an overhead mechanical stirrer and the second aqueous phase was then added. The reaction was heated to 55°C under constant mechanical agitation (150 rpm) for 2h. Then, a third solution of hydrazine hydrate (1.2 mL) in DI water (8 mL) was added dropwise and the mixture was let to react two more hours. The reaction was then cooled down to room temperature, and the capsules were recovered by ultracentrifugation at 35'000 xg. Washing was performed by re-suspending the capsules in 1 % aqueous Pluronic F68 solution and the centrifugation step was repeated two times. Finally, the capsules were re-suspended in DI water for storage.

### Example 4 Synthesis of microcapsules (TDI / PEG800 / tris(aminoethyl) amine)

An organic phase was first formed by mixing a complex perfume composition "Atlantic Santal" (12 mL) and toluene diisocyanate (2.73 mL). A first aqueous phase was prepared from deionized (DI) water (100 mL) and PVA (Mowiol 4-88, 1.68 g). A miniemulsion was prepared by mixing these two solutions, emulsifying with a high-shear homogeneizer at 15'000 rpm for 3 min and ultrasonication (70% amplitude, pulsed, 15 min). In parallel, a second aqueous phase made from DI water (40 mL), PEG800 (4.1 mL), tris(aminoethyl) amine (294 mg) and dibutyltin dilaurate (0.4 mL) was prepared separately. The miniemulsion was transferred to a 250 mL two-necks round bottom flask equipped with an overhead mechanical stirrer and the second aqueous phase was then added to it. The reaction was heated to 55 °C under constant mechanical agitation (150 rpm) for 2h. Then, a third solution of ethylene diamine (1.4 mL) in DI water (8 mL) was added dropwise and the mixture was let to react two more hours. The reaction was then cooled down to room temperature, and the capsules were recovered by ultracentrifugation at 35'000 xg. Washing was performed by resuspending the capsules in 1 % aqueous Pluronic F68 solution and the centrifugation step was repeated two times. Finally, the capsules were resuspended in DI water for storage.

### Example 5 Synthesis of microcapsules (MDI/PEG600/ branched polyethyleneimine)

An organic phase was first formed by mixing limonene (12 mL) and methylene diphenyl diisocyanate (3.86 mL). A first aqueous phase was prepared from deionized (DI) water (100 mL) and PVA (Mowiol 4-88, 1.68 g). A miniemulsion was prepared by mixing these two solutions, emulsifying with a high-shear homogeneizer at 12'000 rpm for 3 min and ultrasonication (80% amplitude, pulsed, 12 min). In parallel, a second aqueous phase made from DI water (40 mL), PEG600 (12.2 mL), branched polyethyleneimine (300 mg) and DABCO (280 mg) was prepared separately. The miniemulsion was transferred to a 250 mL two-necks round bottom flask equipped with an overhead mechanical stirrer and the second aqueous phase was then added to it. The reaction was heated to 60 °C under constant mechanical agitation (150 rpm) for 2h. Then, a third solution of hydrazine hydrate (1.2 mL) in DI water (8 mL) was added dropwise and the mixture was let to react two more hours. The reaction was then cooled down to room temperature, and the capsules were recovered by ultracentrifugation at 35'000 xg. Washing was performed by resuspending the capsules in 1 % aqueous Pluronic F68 solution and the centrifugation step was repeated two times. Finally, the capsules were re-suspended in DI water for storage.

Additional Experimental data can be taken from figs. below.

## Claims

1. A capsule composition comprising a plurality of poly (urethane-urea) microcapsules ("PUU-microcapsules"), wherein
the PUU microcapsules contain an encapsulated hydrophobic active ingredient and a poly (urethane-urea) wall ("PUU-wall"); and
the PUU-microcapsules have a particle size of 20 - 1500 nm and an unimodal size distribution as determined by dynamic light scattering.

2. The composition of claim 1 where said active ingredient is selected from the group consisting of fragrances, malodour counteracting agents, repellents, fungicides, bactericides, pharmaceutically active ingredients, cosmetics, food additives; particularly fragrances.

3. The composition of claim 1 or 2 where said PUU wall comprises the reaction product of
• at least one at least one diisocyanate, preferably selected from the group consisting of aromatic diisocyanates and aliphatic diisocyanates, and
• at least one polyol, preferably selected from the group consisting of polyethylene glycols and cyclic diols (e.g. isosorbide), and
• at least one crosslinker, preferably selected from the group consisting of triols, tetraols and polyamines, and
• at least one curing agent, preferably selected from the group of polyamines, preferably diamines (e.g. hydrazine or ethylenediamine).

4. The composition according to any of the preceding claims, wherein said particle size is 50 - 1000 nm and the particle size distribution is unimodal with a FWHM of 100 - 800 nm.

5. The composition according to any of the preceding claims, wherein said PUU microcapsules have a residual -NCO monomer content < 0.1 %.

6. The composition according to any of the preceding claims in the form of a slurry, said slurry preferably comprising PUU microcapsules as defined in any of claims 1 - 5, water, surfactant and/or stabilizing agents.

7. The composition according to any of the preceding claims in the form of a dried powder.

8. A process for preparing a capsule composition according to any of claims 1 - 7, said process comprising the steps of
(a) preparing the following starting materials:
• an oil phase comprising a hydrophobic active ingredient, a diisocyanate monomer and optionally a diluent ("oil phase");
• a 1^{st} aqueous phase, not miscible with said organic phase, comprising water and a stabilizer;
• a 2^{nd} aqueous phase comprising water, a polyol, a crosslinker and a catalyst;
• a 3^{rd} aqueous phase, comprising water and a curing agent;
(b) combining oil phase and 1^{st} aqueous phase to obtain an oil-in-water dispersion whit droplet size of more than 1500 nm followed by ultrasonic treatment to obtain an oil-in-water emulsion with droplet size of less than 1500 nm;
(c) combining the thus obtained oil-in-water emulsion with said 2^{nd} aqueous phase and applying conditions sufficient to induce interfacial polymerisation to obtain an initial capsule composition in form of a slurry (said capsules having an encapsulated active ingredient and a particles size of 20 - 1500 nm and unimodal size distribution);
(d) combining the thus obtained initial capsule composition with said 3^{rd} aqueous phase under conditions sufficient to induce curing of non-reacted isocyanate groups to obtain the capsule composition in the form of an aqueous emulsion.

9. The process of claim 8 further comprising the steps of:
(e) separating the capsule composition from the aqueous dispersing medium
(f) optionally purifying the capsule composition followed by either (g) or (h)
(g) re-suspending the capsule composition in a 5^{th} aqueous phase, preferably water, to obtain a slurry; or
(h) drying the capsule composition to obtain a dried powder.

10. The process according to any of claims 8 to 9, wherein
• in step (a), all starting materials are present in the form of a solution
• in step (b), emulsification is achieved by high shear mixing with more than 10 000 rpm, followed by US treatment with power input of 400 W at more than 50 % amplitude, preferentially more than 70 % amplitude;
• in step (c), polymerisation takes place at temperatures at 40 - 100°C for 0.5-5 hrs;
• instep (d), curing takes place at 40 - 100°C for 0.5-10 hrs;
• in step (e), separation is effected by centrifugation, preferably at 35 000 xg; I
• in step (f), purification is effected by washing with a 4^{th} aqueous composition comprising water and a surfactant, preferably a non-ionic surfactant (Pluronic F68);
• in step (g), re-suspension is effected by mechanical agitation or ultrasonication, preferably by vortexing;
• in step (h), drying is effected by centrifugation, followed by removal of the supernatant.

11. A capsule composition obtainable by, or obtained by, a process according to any of claims 8 to 10.

12. A woven fabric, a non-woven fabric or a knitted fabric impregnated with a capsule composition according to any of claims 1 to 7.

13. The impregnated fabric of claim 12 further comprising a cross-linking polymer.

14. A textile product comprising the fabric of claim 12 or 13.
